Europäisches Patentamt

(19) European Patent Office

Office européen des brevets

(11) Veröffentlichungsnummer: **0 331 626**
**A2**

(12) **EUROPÄISCHE PATENTANMELDUNG**

(21) Anmeldenummer: **89810034.2**

(22) Anmeldetag: **16.01.89**

(51) Int. Cl.⁴: **A 61 B 17/16**

(30) Priorität: **01.03.88 CH 772/88**

(43) Veröffentlichungstag der Anmeldung:
**06.09.89 Patentblatt 89/36**

(84) Benannte Vertragsstaaten: **AT DE FR GB IT**

(71) Anmelder: **GEBRÜDER SULZER AKTIENGESELLSCHAFT**
**Zürcherstrasse 9**
**CH-8401 Winterthur (CH)**

(72) Erfinder: **Frey, Otto Dr.**
**Wallrütistrasse 56**
**CH-8400 Winterthur (CH)**

**Koch, Rudolf**
**Oberdorfstrasse 229**
**CH-8267 Berlingen (CH)**

(54) **Raspelartiges Räuminstrument.**

(57) Das raspelartige Instrument ist mindestens in seinem Arbeitsbereich als Hohlkörper (1) ausgebildet. Dieser besteht aus zwei in Längsrichtung geteilten Hälften (2a, 2b); die Trennung beider Hälften (2a, 2b) erfolgt in der Mittelebene (3). An einem Ende sind die Hälften (2a, 2b) ineinander einhängbar (13, 14), am anderen können sie miteinander verriegelt (9 - 12) werden.

Die Möglichkeit, den Hohlkörper (1) intraoperativ zu öffnen und wieder zusammenzusetzen, erleichtert die Entnahme des in seinem Inneren angesammelten abgeschälten Knochenmaterial; darüberhinaus vereinfacht sie die Reinigung des Innenhohlraumes nach Gebrauch.

Fig. 1

**Beschreibung**

### Raspelartiges Räuminstrument

Die Erfindung betrifft ein raspelartiges Räuminstrument zum Vorbereiten eines Röhrenknochens für den Einsatz eines Implantatschaftes, welches Instrument über seine Arbeitslänge, auf dem Umfang verteilt, eine Vielzahl von Zähnen hat, die beim Einschlagen in Richtung seiner Achse Knochenmaterial abschälen, wobei das Instrument als Hohlkörper ausgebildet ist und jedem Zahn ein Durchbruch durch die Hohlkörperwand zugeordnet ist.

Ein Instrument der vorstehend genannten Art ist gezeigt und beschrieben in der CH-Anmeldung 00039/87-4. Bei ihm wird das abgeschälte Knochengewebe durch Durchbrüche, die jedem Schälzahn zugeordnet sind, in das Innere des Hohlkörpers transportiert und dort gesammelt. In der Praxis hat sich nun gezeigt, dass dieses abgeschälte Material, das sehr häufig zum Füllen des Zwischenraumes zwischen Implantat und Knochen oder als Material für andere Zwecke während der Operation "wiederverwendet" wird, nur mit Mühe aus dem Hohlkörper zurückgewonnen werden kann. Weiterhin bereitet die Reinigung des Instrumentes und seine Vorbereitung für eine Wiederverwendung Schwierigkeiten.

Der Erfindung liegt deshalb die Aufgabe zugrunde, die geschilderten Nachteile der bisherigen Konstruktion zu beseitigen. Dies geschieht mit der vorliegenden Erfindung dadurch, dass der Hohlkörper entlang einer Längsebene in zwei Hälften geteilt ist, die ineinander einhängbar und miteinander verriegelbar sind.

Das neue Instrument lässt sich mit wenigen Handgriffen auch während einer Operation vom sterilen Hilfspersonal öffnen, wonach dann das über die ganze Höhe seiner Arbeitslänge verteilte abgeschälte Knochenmaterial leicht entnommen werden kann. Eine solche Entnahme ist dabei nicht nur am Ende einer Räumung des Knochens, sondern auch zwischendurch möglich, da nicht nur das Oeffnen, sondern auch das Schliessen des Instrumentes auf einfache Weise erfolgen kann.

Das intraoperative Schliessen wird dabei erheblich erleichtert, wenn die beiden Hälften in ihrer genauen Lage zueinander justierbar sind.

Im folgenden wird die Erfindung anhand eines Ausführungsbeispiels im Zusammenhang mit der Zeichnung näher erläutert.

Fig. 1 zeigt schematisch eine Ansicht des neuen Instrumentes, teilweise im Schnitt;

Fig. 2 gibt eine Variante der distalen Einhängung der beiden Instrumentenhälften wieder.

Der Hohlkörper 1 des Instrumentes ist aus zwei U-förmig gebogenen Blechen 2a und 2b gebildet, die sich mit ihren offenen Seiten längs einer Mittelebene 3 gegeneinander abstützen. In ihrem Arbeitsbereich, d.h. in dem Bereich ihrer Höhe, in dem sie aktiv als Raspel wirken und beim Einschlagen in einen Knochenhohlraum Gewebe abschälen, sind sie mit einer Vielzahl von nur schematisch angedeuteten Zähnen 4 bestückt. Jeder Zahn ist mit einer Schneidkante an seinem unteren Rand versehen. Jedem Zahn ist weiterhin je ein Durchbruch 5 durch die Wände des Hohlkörpers 1 zugeordnet, durch den das abgeschälte Gewebe in den Hohlkörper 1 hinein "abfliesst".

Obwohl bei den beiden gezeigten Ausführungsbeispielen der Arbeitsbereich der Raspel sich über ihre ganze Höhe erstreckt, ist es auch möglich - wie bei der erwähnten bekannten Hohlkörper-Raspel - nur einen Teil der Raspelhöhe als Arbeitsbereich auszugestalten, an den nach distal beispielsweise ein glattes zahnfreies Führungselement angesetzt ist.

An jeder der beiden Hohlkörperhälfte 2a und 2b ist am proximalen Ende ein massiver Klotz 6a und 6b angesetzt, der im gezeigten Beispiel angeschweisst ist. Der eine Klotz 6a enthält einen Positionierstift 7, während in dem anderen 6b eine entsprechende Gegenbohrung 8 vorgesehen ist. Mit den beiden Elementen 7 und 8 wird eine gegenseitige Justierung der beiden Hälften 2a und 2b beim Zusammenbau der Raspel gewährleistet.

Im zusammengebauten Zustand bilden die beiden Klötze 6a und 6b ein im Querschnitt T-förmiges Kopfende 9 mit horizontalen Nuten 10. In dieses Kopfende 9 kann das krallenförmige Ende 11 eines massiven Oberteils 12 horizontal eingeschoben werden, durch das die beiden Hälften 2a und 2b miteinander verriegelt werden. Zwischen dem Oberteil 12 und dem Kopfende 9 kann dabei zusätzlich eine Sicherung, beispielsweise in Form eines federnd abgestützten Sicherungsstiftes, gegen unbeabsichtigtes Lösen ihrer Verbindung vorgesehen sein, was nicht ausdrücklich dargestellt ist.

Das Oberteil 12 endet in einem nicht dargestellten Amboss für ein Einschlaginstrument. Amboss und Oberteil sind dabei so geformt, dass die Kraftwirkung der Schläge in Richtung der Mittelebene und Längsachse 3 erfolgen.

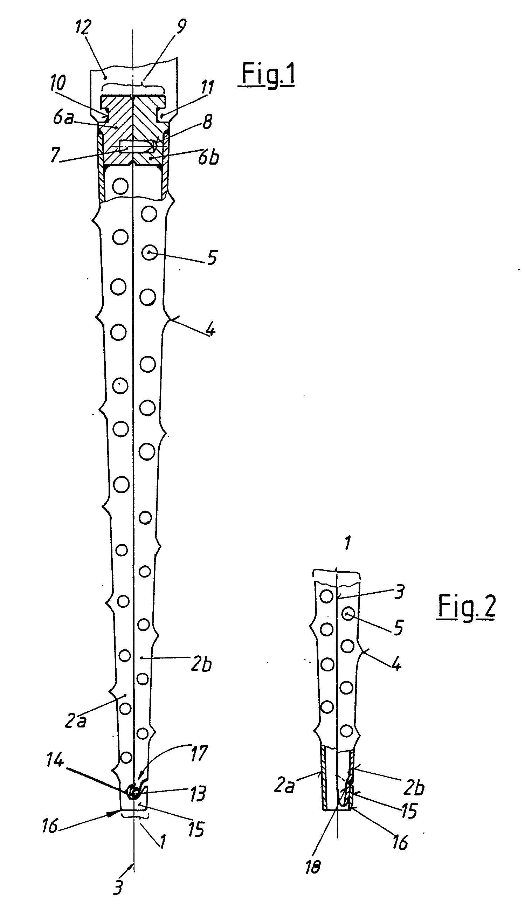

Das Einhängen der beiden Hälfte 2a und 2b erfolgt an ihrem distalen Ende. In Fig. 1 endet dafür die Hälfte 2b in sich über die Mittelebene 3 hinaus erstreckenden Lappen, in denen nach aussen weisende Lagerzapfen 13 vorgesehen sind. Als Gegenlager für diese Zapfen 13 dient eine in der Mittelebene 3 liegende Lagerschale 14 der Hohlkörperhälfte 2a. Diese ist daher an ihrem Ende als ein den geschlossenen Umfangs des Hohlkörpers 1 bildender "Ring" 15 ausgebildet, der gegebenenfalls in einer scharfen Schneidkante 16 enden kann. Durch einen schräg nach rechts oben verlaufenden Schlitz 17, dessen Breite mindestens dem Druchmesser des Zapfens 13 entspricht, können die Zapfen 13 in die Lagerschalen 14 eingesetzt werden.

Eine etwas einfachere Konstruktion der Einhängung zeigt Fig. 2; hier endet die Hälfte 2b in einer etwas in den Hohlraum hinein versetzten Zunge 18, die in den in Umfangsrichtung geschlossenen Ring 15 einschiebbar ist und sich an dessen "Rückwand" anlegt.

Selbstverständlich sind sowohl die Verriegelung als auch die Einhängvorrichtungen für die beiden Hälften 2a und 2b nicht auf die gezeigten Beispiele beschränkt, sondern können durch andere bekannte Konstruktionselemente ersetzt werden.

**Patentansprüche**

1. Raspelartiges Räuminstrument zum Vorbereiten eines Röhrenknochens für den Einsatz eines Implantatschaftes, welches Instrument ber seine Arbeitslänge, auf dem Umfang verteilt, eine Vielzahl von Zähnen hat, die beim Einschlagen in Richtung seiner Achse Knochenmaterial abschälen, wobei das Instrument als Hohlkörper ausgebildet ist und jedem Zahn ein Durchbruch durch die Hohlkörperwand zugeordnet ist, **dadurch gekennzeichnet, dass** der Hohlkörper (1) entlang einer Längsebene (3) in zwei Hälften (2a,2b) geteilt ist, die ineinander einhängbar und miteinander verriegelbar sind.

2. Räuminstrument nach Anspruch 1, **dadurch gekennzeichnet, dass** die beiden Hälften (2a,2b) in ihrer genauen Lage zueinander justierbar sind.

Fig.1

Fig.2